## Europäisches Patentamt

## European Patent Office

## Office européen des brevets

(11) Veröffentlichungsnummer: **0 058 903**
**A1**

(12) **EUROPÄISCHE PATENTANMELDUNG**

(21) Anmeldenummer: 82101088.1

(22) Anmeldetag: 15.02.82

(51) Int. Cl.³: **A 01 N 1/02**, A 61 L 2/18,
F 26 B 5/06, A 61 K 35/16

(30) Priorität: 19.02.81 DE 3106073

(43) Veröffentlichungstag der Anmeldung: 01.09.82
Patentblatt 82/35

(84) Benannte Vertragsstaaten: AT BE CH DE FR GB IT LI LU NL SE

(71) Anmelder: BOEHRINGER MANNHEIM GMBH,
Sandhofer Strasse 112-132 Postfach 31 01 20,
D-6800 Mannheim 31-Waldhof (DE)

(72) Erfinder: Woog, Heinrich, Dr. rer. nat., Lindenstrasse 6,
D-6941 Laudenbach (DE)
Erfinder: Gruber, Werner, Am Heiligenberg 12,
D-6943 Birkenau (DE)
Erfinder: Osmers, Knut, Dr. rer. nat., Ringweg 1a,
D-6940 Weinheim (DE)

(54) Verfahren zur Herstellung von sterilen Zubereitungen hochviskoser Lösungen und Substanzen.

(57) Es wird ein neues Verfahren zur Herstellung steriler Zubereitungen von Stoffen beschrieben, die herkömmlichen Methoden der Sterilisation entweder nicht zugänglich sind oder durch diese unerwünscht verändert werden. Das Verfahren zeichnet sich dadurch aus, dass man ein Sterilisationsmittel unter schonenden Bedingungen einwirken lässt, dieses dann zusammen mit dem Lösungsmittel bei einer anschliessenden Lyophilisation oder Sprühtrocknung annähernd quantitativ wieder entfernt und das Produkt gegebenenfalls durch Zusätze in eine pharmazeutisch akzeptable bzw. zur Anwendung an isolierten tierischen oder menschlichen Zellen oder Zellkulturen geeignete Form überführt.

EP 0 058 903 A1

Verfahren zur Herstellung von sterilen Zubereitungen
hochviskoser Lösungen und Substanzen

Die vorliegende Erfindung betrifft Verfahren zur Herstellung steriler Zubereitungen von Stoffen, die herkömmlichen Methoden der Sterilisation entweder nicht zugänglich sind oder durch diese unerwünscht verändert werden. Die Verfahren zeichnen sich dadurch aus, daß man ein Sterilisationsmittel unter schonenden Bedingungen einwirken läßt, dieses dann zusammen mit dem Lösungsmittel bei einer anschließenden Lyophilisation oder Sprühtrocknung annähernd quantitativ wieder entfernt und das Produkt gegebenenfalls durch Zusätze in eine pharmazeutisch akzeptable bzw. zur Anwendung an isolierten tierischen oder menschlichen Zellen oder Zellkulturen geeignete Form überführt.

Zur Keimfreimachung von Substanzen und Lösungen von Substanzen sind unter anderem folgende Methoden üblich:

- Zusatz von Konservierungsmitteln,
- Hitzesterilisation und
- Sterilfiltration.

Bei der Herstellung von sterilen Lösungen hochviskoser Stoffe treten insbesondere dann Probleme auf, wenn

- durch Zugabe von Konservierungsmitteln eine Interferenz zwischen Konservierungsmittel und Wirkstoff erfolgt oder die allergisierende Komponente der Konservierungsmittel negative Nebenwirkungen hervorruft,

- der Wirkstoff durch Hitzesterilisation unvorteilhaft verändert wird oder

- das zu sterilisierende Substrat aufgrund seiner Molekülgröße und/oder Viskosität nicht mehr über Membranfilter sterilfiltriert werden kann.

Als besonders schwierig hat sich die Gewinnung von sterilen Lösungen des Blutplasmas oder steriler Fraktionen des Blutplasmas, verschiedener hochmolekularer Nucleinsäuren, wie Desoxyribonucleinsäure, Ribonucleinsäure sowie auch von langkettigen Polynucleotid-Homopolymeren, z.B. von Polyinosinsäure-Polycytidylsäurekomplexen (Poly I : C) oder Polyadenylsäure-Polyuridinylsäurekomplexen (Poly A : U) erwiesen.

Unter Poly I : C versteht man einen zweistrangigen Komplex aus Polyinosinsäure und Polycytidylsäure, welcher als Auslöser für die Bildung von Interferon ermittelt wurde und deshalb als Interferoninducer bezeichnet wird (s. hierzu z.B. US-PS 4.124.702 und DE-OS 20 10 734).

Derartige Komplexe werden erhalten, wenn man zueinander komplementäre Homopolynucleotide unter bestimmten Bedingungen (z.B. Enzym-Katalyse, Ultraschall usw.) in Lösung vereinigt. Dabei lagern sich zum Beispiel ein Polyinosinsäure-Strang (Poly I) und ein Polycytidylsäure-Strang (Poly C) unter Ausbildung von Wasserstoffbrückenbindungen zwischen den Basen zu einem Doppelstrang und diese wiederum aufgrund von "Stapelungskräften" zu einer doppelhelicalen Tertiärstruktur von Poly I : C zusammen.

0058903

Erhöht man die Temperatur einer Lösung von Poly I : C
so erreicht man schließlich eine Temperatur, bei der die
Struktur des Doppelstranges auseinanderbricht.
In der Lösung liegen nun die getrennten ("denaturierten")
Einzelstränge vor. Man sagt, der Doppelstrang ist geschmolzen und nennt die Temperatur, bei der dies stattfindet, die Schmelztemperatur. Kühlt man die Lösung,
die nun die aufgeschmolzenen Einzelstränge enthält,
langsam ab, so tritt wieder Doppelstrangbildung ("Renaturierung") ein.

Am Schmelzvorgang sind auch die sich abstoßenden Ladungen der Phosphatgruppen beteiligt. Durch
hohe Salzkonzentrationen kann man diese Abstoßung abschirmen. Daher wurden früher Lösungen von Poly I : C
hergestellt, indem man Lösungen von Poly I und Poly C
in Gegenwart einer Salzlösung, z.B. 0,1 Mol Calciumchloridlösung bei einem pH-Wert von etwa 7 bei Raumtemperatur oder unter geringem Erwärmen miteinander mischte.
Diese Lösung ließ man 1-2 Wochen stehen. In dieser Zeit
bildete sich eine gelartige nichtsterile viskose Masse.

Sterile Poly I : C-Lösungen werden erstmals nach
DE-OS 20 35 790 gewonnen, indem man Poly I und Poly C
jeweils getrennt in Gegenwart einer gepufferten Salzlösung löst, die getrennten Lösungen unter Bildung von
gelatinösen, trüben Komplexen vereinigt, die Komplexe
im Autoklaven bis 135°C erhitzt und danach langsam
abkühlen läßt.

Untersuchungen haben gezeigt, daß nach dem obigen Verfahren hergestellte sterile Poly I : C-Lösungen offenbar
aufgrund der starken thermischen Belastung nicht mehr
die ursprüngliche Kettenlänge aufweisen und eine geringere
interferoninducierende Wirkung entfalten.

0058903

Die dazu erforderlichen Ergebnisse erhält man auf die folgende Weise: Schmilzt man einen Doppelstrang aus Poly I · Poly C auf, so tritt eine Zunahme der Extinktion bei 260 nm ein. Man erhält eine Schmelzkurve, bei der als Abszisse die Temperatur und als Ordinate die Extinktion aufgetragen wird. Der Schmelzvorgang verläuft kooperativ. Aus der Theorie solcher kooperativer Prozesse folgt, daß bei hohem Molekulargewicht der Doppelstränge eine steile Schmelzkurve erhalten wird. Je kleiner die Moleküle sind, desto eher beginnt der Schmelzvorgang und desto flacher verläuft die Schmelzkurve. Eine quantitative Vorstellung, wie hoch das mittlere Molekulargewicht ist, erhält man, wenn man die Sedimentationskonstante in der analytischen Ultrazentrifuge bestimmt.

Diese Sedimentationskonstanten werden in S ($\triangleq$ Svedberg-Einheiten) angegeben und betragen für erfindungsgemäß sterilisiertes Poly I : C 12-15, während Material, das gemäß DE-OS 20 35 790 sterilisiert wird, Werte unterhalb 10 ergibt.

Da zudem, wie bereits oben erwähnt, weder die einzelnen Polynucleotide noch die langkettigen Polynucleotidkomplexe wegen Ihrer Sekundär- und Tertiär-Struktur über übliche Membranfilter sterilfiltriert werden können, mußte nach anderen Sterilisationsmethoden gesucht werden, die bei äußerst schonender Sterilisation das Blutplasma und die o.g. Nucleinsäuren nicht zersetzen, möglichst lange Ketten der Homopolymeren erhalten und somit eine höhere interferoninduzierende Wirkung des Poly I : C-Komplexes erbringen.

Diese Aufgabe wird überraschend einfach dadurch gelöst, daß man unter relativ milden Bedingungen mit einem Konservierungsmittel die Keime abtötet und einen Lyophilisationsprozeß oder Sprühtrocknungsprozeß anschließt, in dessen Verlauf neben dem Lösungsmittel auch das Konservierungsmittel weitestgehend wegsublimiert oder verdampft.

- 5 -

0058903

Das erfindungsgemäße Prinzip ist selbstverständlich nicht auf den oben geschilderten Spezialfall der Sterilisation von Poly I : C-Lösungen beschränkt, sondern kann in vielfältiger Weise zur Entkeimung von hochviskosen Lösungen und Substanzen eingesetzt werden.

Gegenstand der Erfindung sind daher Verfahren zur Herstellung von sterilen Zubereitungen hochviskoser Lösungen und Substanzen, wie zum Beispiel von Blutplasma oder Fraktionen des Blutplasmas, von verschiedenen hochmolekularen Nucleinsäuren, wie Desoxyribonucleinsäure, Ribonucleinsäure sowie auch von langkettigen Polynucleotid-Homopolymeren und von Komplexen solcher Polymeren, die sich dadurch auszeichnen, daß man mit einem Konservierungsmittel die Keime abtötet, das Konservierungsmittel im Verlaufe eines anschließenden Lyophilisations- oder Sprühtrocknungsprozesses zusammen mit dem Lösungsmittel weitestgehend wegsublimiert oder abdampft und gegebenenfalls durch Zugabe geeigneter üblicher Hilfsmittel eine pharmazeutisch akzeptable Form herstellt.

Unter Zubereitungen werden im Sinne der Erfindung alle Zubereitungsformen von Stoffen verständen, die unmittelbar oder mittelbar zur pharmazeutischen Anwendung oder zur Anwendung an isolierten tierischen oder menschlichen Zellen bzw. Zellkulturen geeignet sind. Dies sind zum Beispiel Lösungen, Suspensionen, Salben, Granulate, Pulver oder Tabletten.

Als Konservierungsmittel eignen sich alle Stoffe, die bei der Behandlung des Blutplasmas, der Nucleinsäuren und der Polynucleotid-Homopolymeren gemäß unserem Verfahren die entsprechende bakterizide Wirkung aufzeigen, dabei die Struktur der Wirkstoffe und die Aktivität der Polynucleotide während des Sterilisationsvorganges nicht beeinträchtigen und beim Lyophilisieren oder Sprühtrocknen aus der Lösung sublimieren bzw. verdampfen.
Die Konservierungsmittel müssen während der Lyophilisation oder Sprühtrocknung eliminiert werden, da diese
- z.B. die interferoninduzierende Aktivität bei
Poly I : C - oder eine andere erwünschte Eigenschaft
beeinträchtigen können. Solche Konservierungsmittel
sind zum Beispiel Chloreton, Benzylalkohol, p-Chlor-m-cresol
oder Pyrokohlensäure-diethylester. Bevorzugt sind Chloreton
oder Pyrokohlensäure-diethylester.

Die Konzentration der zu sterilisierenden Komponenten
beträgt bei den Polynucleotid-Komplexen vor der Lyophilisation max. 0,3 %, bevorzugt 0,1 %, bei der Desoxyribonucleinsäure und Ribonucleinsäure max. 2 %, bevorzugt
0,1 %.

Die Konzentration der verschiedenen Konservierungsmittel
hängt von der Konzentration der Wirkstoffe ab und kann
von Fall zu Fall nach dem Fachmann geläufigen Verfahren
bestimmt werden.

Eine Ausgestaltung des erfindungsgemäßen Verfahrens
besteht darin, daß man Blutplasma, Nucleinsäuren oder
Polynucleotid-Homopolymere bzw. Komplexe aus solchen
Polymeren, gegebenenfalls in Anwesenheiten von Puffersubstanzen, löst, zu diesen Lösungen die erforderlichen
Mengen Konservierungsmittel zugibt und, falls notwendig,
erwärmt. Man läßt das Konservierungsmittel bzw. Desinfektionsmittel 10 Min. - 4 Std. (bevorzugt 30 Min. -
2 Std.) bei 25-100°C (bevorzugt bei 40-80°C) einwirken.
Danach wird die Wirkstofflösung lyophilisiert oder sprühgetrocknet, wobei das Konservierungsmittel während der
ation oder während der Sprühtrocknung wegsublimiert
oder verdampft. Die erfindungsgemäßen Lyophilisate und sprühgetrockneten Granulate sind ohne weiteres schnell in Wasser löslich und ergeben eine sterile Lösung.

Eine bevorzugte Ausgestaltung des erfindungsgemäßen Verfahrens ist die Herstellung von sterilen Zubereitungen von Polyinosinsäure-Polycytidylsäure-Komplexen (Poly I : C), Polyadenylsäure-Polyuridinsäure-Komplexen (Poly A : U), Polyadenylsäure-Cytidylsäure (Poly A : C), Polyadenylsäure-Guanylsäure-Uridylsäure (Poly A : G : U), Thionicotinamid-adenin-dinucleotidphosphat (Thio-NADP) oder 2-Desoxy-adenosin-5'-triphosphat (dATP) (Dinatriumsalz) sowie sterilen Konzentraten von t-Ribonucleinsäure oder Blutplasma.

Überraschenderweise konnte festgestellt werden, daß die nach dem erfindungsgemäßen Verfahren hergestellten konzentrierten sterilen Polynucleotid-Homopolymer-Komplexe einen höheren interferoninduzierenden Effekt hervorrufen.

Erstaunlich hierbei ist die Tatsache, daß nach einer sehr kurzen Einwirkungszeit der Konservierungsmittel eine vollständige Abtötung der Keime erfolgt , so daß in einem weiteren Arbeitsvorgang (Lyophilisation oder Sprühtrocknung) das nicht mehr benötigte Konservierungsmittel nahezu vollständig entfernt werden kann, denn normalerweise werden Konservierungsmittel eingesetzt, um das Keimwachstum zu hemmen, nicht aber um die Keime vollständig abzutöten.

Man erhält, wie nachträglich festgestellt wurde, z.B. bei Poly I : C wesentlich längere Ketten der Homopolymeren und somit eine höhere interferoninduzierende Wirkung. Die nach unserem Verfahren gewonnenen Homopolymeren-Komplexe sind durch den Lyophilisationsprozeß oder durch die Sprühtrocknung von dem flüchtigen Konservierungsmittel weitestgehend befreit. Dies ist ein wesentlicher Faktor, da die Konservierungsmittel die interferoninduzierende Wirkung der Polynucleotide einschränken.

- 8 -

0058903

Ein weiterer Gegenstand der Erfindung sind daher pharmazeutische oder zur Anwendung an isolierten tierischen oder menschlichen Zellen bzw. Zellkulturen geeignete Zubereitungen, die die erfindungsgemäß sterilisierten Zubereitungen hochviskoser Lösungen und Substanzen enthalten.

Die folgenden Beispiele sollen den Gegenstand der Erfindung näher erläutern, ihn aber in keiner Weise einschränken:

Beispiel 1

<u>Steriles Poly I : C-Lyophilisat</u>

Sämtliche Glasgeräte, die mit der Poly I : C-Lösung
in Berührung kommen, werden sorgfältig mit Chromschwefelsäure gereinigt und anschließend gut mit Wasser für
Injektionszwecke nachgespült.

In einem wie oben beschriebenen gereinigten 10-12-1-
Glaskolben mit Rückflußkühler und Rührmotor werden 9 1
Wasser für Injektionszwecke vorgelegt und darin unter
Erwärmen auf 50-60$^{\circ}$C 100 g Poly I : C mit sogenanntem
Interferonpuffer gelöst.

In diesen 100 g des o.g. Gemisches befinden sich 10 g
Poly I : C sowie 90 g Interferonpuffer (bestehend aus
0,006 M $Na_3PO_4 \cdot 12\ H_2O$ und 0,15 M NaCl, eingestellt auf
pH 7,0). Nach dem Lösen des Feststoffes wird zum Endvolumen von 10 1 mit Wasser aufgefüllt. In die fertige
Lösung wird zum Endvolumen von 10 1 mit Wasser aufgefüllt. In die fertige Lösung werden 50 g Chloreton gegeben
und 1 Stunde bei 80$^{\circ}$C am Rückfluß gerührt. Die Lösung
kühlt man auf Raumtemperatur ab. Nun wird die Lösung
sprühgetrocknet oder zu 5 ml in sterile Ampullenflaschen
unter aseptischen Bedingungen abgefüllt und unter den
vorher als optimal ermittelten Bedingungen lyophilisiert.

Beispiel 2

Steriles Poly A : U-Lyophilisat

Wie im Beispiel 1 beschrieben, werden alle zur Herstellung notwendigen Geräte gereinigt. Danach werden in 2,9 l Wasser für Injektionszwecke 31,2 g Poly A : U gelöst.

Es werden 14,5 g Chloreton dazugegeben und 1 Std. bei 80°C unter Rückfluß und Rühren erwärmt. Nach dem Abkühlen auf Raumtemperatur wird die Lösung sprühgetrocknet. Auch kann man die gekühlte Lösung zu 5,1 ml in die sterilisierten Ampullenflaschen füllen und unter den vorher als optimal ermittelten Bedingungen lyophilisieren.

Beispiel 3

Steriles Blutplasma

500 ml Blutplasma werden mit 2,5 g Chloreton gemischt und 15 Min. auf 35°C erwärmt. Die Lösung kühlt man auf Raumtemperatur ab und lyophilisiert unter den vorher als optimal ermittelten Bedingungen.

Beispiel 4

Steriles t-RNA-Lyophilisat oder Sprühgranulat

500 mg Transfer-Ribonucleinsäure (t-RNA) werden in 500 ml 1,1-%iger Interferonpuffer-Lösung gelöst und unter Zugabe von 5,0 g Pyrokohlensäurediethylester 1 Std. auf 60°C erwärmt. Danach wird die Lösung unter ermittelten Bedingungen lyophilisiert oder sprühgetrocknet.

Beispiel 5

Sterile Polyadenyl-Cytidylsäure (Poly A:C)

500 mg Polyadenyl-Cytidylsäure (Poly A : C ) werden in
500 ml 1,1 %iger Interferonpufferlösung gelöst und
unter Zugabe von 5,0 g Chloreton 1 Stunde auf 60$^o$C
erwärmt.

Anschließend wird die Lösung unter ermittelten Bedingungen
lyophilisiert oder sprühgetrocknet.


Beispiel 6

Sterile Polyadenyl-Guanyl-Uridylsäure (Poly A:G:U)

500 mg Polyadenyl-Guanyl-Uridylsäure (Poly A : G : U )
werden in 500 ml 1,1 %iger Interferonpufferlösung gelöst
und unter Zugabe von 5,0 g Chloreton 1 Std. auf 60$^o$C
erwärmt.

Danach wird die Lösung unter ermittelten Bedingungen lyophilisiert oder sprühgetrocknet.


Beispiel 7

Steriles Thionicotinamid-adenin-dinucleotidphosphat (Thio-NADP)

1,0 g Thionicotinamid-adenin-dinucleotidphoshat (Thio-NADP)
werden in 500 ml Wasser gelöst und unter Zugabe von 5,0 g
Pyrokohlensäurediäthylester 1 Std. auf 60$^o$C erwärmt. Anschließend wird die Lösung unter den ermittelten Bedingungen lyophilisiert oder sprühgetrocknet.

Beispiel 8

**Steriles 2-Desoxy-adenosin-5'-triphosphat (dATP)(Dinatriumsalz)**

1,0 g 2-Desoxy-adenosin-5'-triphosphat (dATP) (Dinatriumsalz) werden in 500 ml Wasser gelöst und unter Zugabe von 5,0 g Pyrokohlensäurediäthylester 1 Std. auf 60°C erwärmt. Anschließend wird die Lösung unter den ermittelten Bedingungen lyophilisiert oder sprühgetrocknet.

Bei allen Fällen ergab sich nach Wiederauflösen der so gewonnenen Lyophilisate eine keimarme, hochviskose Lösung mit hoher interferoninduzierender Wirkung (letzteres gilt nicht für Beispiel 3).

0058903

Patentansprüche

1. Verfahren zur Herstellung steriler Zubereitungen hochviskoser Lösungen und Substanzen, dadurch gekennzeichnet, daß man ein Konservierungs- bzw. Desinfektionsmittel einwirken läßt, anschließend lyophilisiert oder sprühtrocknet und gegebenenfalls anschließend durch Zugabe üblicher steriler Hilfsmittel eine pharmazeutisch akzeptable bzw. zur Anwendung an isolierten tierischen oder menschlichen Zellen oder Zellkulturen geeignete Form überführt.

2. Verfahren zur Herstellung steriler Zubereitungen des Blutplasmas oder steriler Fraktionen des Blutplasmas, verschiedener hochmolekularer Nucleinsäuren sowie von langkettigen Polynucleotid-Homopolymeren bzw. von Polynucleotid-Homopolymer-Komplexen, dadurch gekennzeichnet, daß man ein Konservierungs- bzw. Desinfektionsmittel einwirken läßt, anschließend lyophilisiert oder sprühtrocknet und gegebenenfalls anschließend durch Zugabe üblicher steriler Hilfsmittel eine pharmazeutisch akzeptable bzw. zur Anwendung an isolierten tierischen oder menschlichen Zellen oder Zellkulturen geeignete Form überführt.

3. Verfahren zur Herstellung steriler Zubereitungen von Blutplasma, t-Ribonucleinsäure, Polyinosinsäure-Polycytidylsäure-Komplexen (Poly I : C), Polyadenylsäure-Polyuridinsäure-Komplexen (Poly A : U), Polyadenylsäure-Cytidylsäure (Poly A : C), Polyadenylsäure-Guanylsäure-Uridylsäure (Poly A : G : U), Thionicotinamid-adenin-dinucleotidphosphat (Thio-NADP) oder 2-Desoxy-adenosin-5'-triphosphat (dATP) (Dinatriumsalz), dadurch gekennzeichnet, daß man ein Konservierungs- bzw. Desinfektionsmittel einwirken läßt, anschließend lyophilisiert oder sprühtrocknet und gegebenenfalls anschließend durch Zugabe üblicher steriler Hilfsmittel eine pharmazeutisch akzeptable bzw. zur Anwendung an isolierten tierischen oder menschlichen Zellen oder Zellkulturen geeignete Form überführt.

0058903

4. Verfahren nach einem der Ansprüche 1-3, dadurch gekennzeichnet, daß das Konservierungs- bzw. Desinfektionsmittel aus der Gruppe Chloreton, Benzylalkohol, p-Chlor-m-cresol und Pyrokohlensäure-diethylester ausgewählt ist.

5. Verfahren nach Anspruch 4, dadurch gekennzeichnet, daß das Konservierungs- bzw. Desinfektionsmittel Chloreton oder Pyrokohlensäure-diethylester ist.

6. Verfahren nach einem der Ansprüche 1-5, dadurch gekennzeichnet, daß das Konservierungs- bzw. Desinfektionsmittel 10 Min. - 4 Std. bei 25-100$^{\circ}$C einwirkt.

7. Verfahren nach Anspruch 6, dadurch gekennzeichnet, daß das Konservierungs- bzw. Desinfektionsmittel 30 Min. - 2 Std. bei 40-80$^{\circ}$C einwirkt.

8. Pharmazeutische Zubereitungen enthaltend sterile Zubereitungen hochviskoser Lösungen oder Substanzen, die nach einem der Ansprüche 1-7 hergestellt werden.

9. Zur Anwendung an isolierten tierischen oder menschlichen Zellen bzw. Zellkulturen geeignete Zubereitungen enthaltend sterile Zubereitungen hochviskoser Lösungen oder Substanzen, die nach einem der Ansprüche 1-7 hergestellt werden.

10. Zur pharmazeutischen Anwendung oder zur Anwendung an isolierten tierischen oder menschlichen Zellen bzw. Zellkulturen geeignete Zubereitungen enthaltend sterile Zubereitungen von Poly I : C, die nach Anspruch 7 hergestellt werden.

## EUROPÄISCHER RECHERCHENBERICHT

### Europäisches Patentamt

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| | "Keine Entgegenhaltungen" ----- | | A 01 N 1/02<br>A 61 L 2/18<br>F 26 B 5/06<br>A 61 K 35/16 |

| | RECHERCHIERTE SACHGEBIETE (Int. Cl. 3) |
|---|---|
| | A 01 N<br>A 61 K<br>A 61 L<br>F 26 B |

Der vorliegende Recherchenbericht wurde für alle Patentansprüche erstellt.

| Recherchenort | Abschlußdatum der Recherche | Prüfer |
|---|---|---|
| DEN HAAG | 18-05-1982 | MALHERBE Y.J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82